# EUROPEAN PATENT APPLICATION

(11) **EP 4 579 231 A1**
(43) Date of publication of application: **02.07.2025**
(21) Application number: 23834996.3
(22) Date of filing: 07.07.2023
(51) Int. Cl.: G01N 27/48, G01N 33/18

(54) **ELECTROCHEMICAL METHOD FOR DETECTING, IDENTIFYING AND QUANTIFYING ANALYTES, AND EQUIPMENT FOR CARRYING OUT SAID METHOD**

(30) Priority: 07.07.2022 ES 202230625
(71) Applicant: Blueming S.L.U., 30730 San Javier Murcia (ES)
(72) Inventor: HERENA GARCÍA, Rafael, 30730 SAN JAVIER (Murcia) (ES)
(74) Representative: Díaz Pacheco, Maria Desamparados
(86) International application number: PCT/ES2023/070440
(87) International publication number: WO 2024/008992

(57) **Abstract**

An electrochemical method for detecting, identifying, and quantifying ions and nutrients dissolved in water through voltammetry, and equipment to carry out said method, comprising: a preparation/placement stage of a sensor (3), with a reference electrode (3a), working electrode (3b), and counter electrode (3c), in a potentiostat (2); a stage for contacting a sample or analysis solution containing the analyte with the sensor (3); a stage for applying, controlled via software from a computer system (4), electrical stimulation to the sample through Cyclic Voltammetry, Normal Pulse Voltammetry, Differential Pulse Voltammetry, or Square Wave Voltammetry, with a step potential and pulse amplitude range between - 3,000 mV and 3,000 mV; and a stage for characterizing one or more analytes based on at least one measurement of the signals resulting from the oxidation-reduction of the analyte(s).

## Description

The invention, as stated in this descriptive report, relates to an electrochemical method for the detection, identification, and quantification of analytes, such as various ions and nutrients dissolved in water through voltammetry, and equipment to carry out said method. This method offers advantages and features detailed below.

The objective of the present invention focuses on an electrochemical method for detecting, identifying, and quantifying ions and nutrients dissolved in water through voltammetry. The method consists of predefined protocols and is based on the interpretation of signals obtained from one or more consecutive electrochemical stimuli at low potentials, utilizing a potentiostat for voltammetric analysis applied to printed electrodes. The signals are interpreted through specifically developed software designed to generate and process them. Another aspect of the invention relates to the measurement equipment required to perform the mentioned detection method, which essentially includes the aforementioned potentiostat, an interconnectable sensor with the electrodes, and a computing system equipped with specific software to control stimulation, read signals, and interpret them, along with a display unit serving as the user interface.

### FIELD OF APPLICATION

The field of application for this invention lies within industries specializing in the physicochemical analysis of water.

### BACKGROUND OF THE INVENTION

A key aspect of water quality analysis, proper maintenance of water resources, wastewater management, aquaculture, and other productive activities is having an appropriate monitoring system for certain water parameters such as pH, temperature, and salinity. Additionally, dissolved residues/nutrients continuously produced by living organisms, including phosphorus-rich chemical species such as phosphates (PO4) and nitrogen-rich species like ammonium (NH4), nitrite (NO2), and nitrate (NO3), must be monitored.

In the environment, the accumulation of these chemicals from human activities like agriculture and livestock farming can lead to eutrophication (over-fertilization) of nearby water bodies, causing associated environmental problems. Regarding the maintenance or cultivation of aquatic organisms, whether in aquaculture or domestic aquariums, these chemical species can be highly toxic to organisms when they reach certain abundance levels. For example, ammonium becomes hazardous at a concentration as low as 1 part per million (ppm). Given that its generation is a direct consequence of the excretion of maintained living organisms, its continuous increase in high-density systems such as aquaculture facilities and private aquariums necessitates monitoring to maintain safe concentration levels.

Unfortunately, existing technologies are often expensive, labor-intensive, and environmentally harmful, motivating the development of the present method.

Currently, both domestically and industrially, colorimetric systems remain the most widely used for determining nutrients in water. These systems involve adding several chemicals (mostly hazardous) to a water sample, producing a color whose intensity correlates with the dissolved quantity of the targeted substance, such as ammonium. This approach is tedious and time-consuming, often requiring 20 to 30 minutes for daily analysis. Other analytical technologies, such as potentiometric systems, are widely used to measure parameters like pH, REDOX potential, and water conductivity. However, the continued reliance on colorimetric systems for measuring dissolved nutrients signals the inadequacies of existing products, which are limited to analyzing a few chemicals, often provide unstable measurements, and are prohibitively expensive. Furthermore, these technologies often require different sensors for analyzing various parameters or chemicals, with replacements being costly.

It is known that patent NL1043265 discloses a method for the electrochemical identification of compounds focused on forensic chemistry, specifically narcotics, through voltammetry. This method involves the step of applying a potential between -3000 mV and 3000 mV to a solution containing the compound and measuring the resulting current. Patent US 6413398 is also known, which discloses a method for the electrochemical identification of compounds, specifically complex molecules such as polypeptides, antibiotics, and pesticides, through voltammetry. This method involves the step of applying a potential between -2000 mV and 2000 mV to a solution containing the compound and measuring the resulting current.

The objective of the present invention is, therefore, to develop an improved method to achieve such measurements much more quickly and practically, avoiding the cited drawbacks of current systems. Additionally, as a reference to the current state of the art, it should be noted that, at least on the part of the applicant, there is no knowledge of any other method presenting technical characteristics identical or similar to those claimed herein.

### EXPLANATION OF THE INVENTION

The electrochemical detection, identification, and quantification method for ions and nutrients dissolved in water through voltammetry proposed by this invention is configured as the optimal solution to the aforementioned objective. The characterizing details that make it possible and conveniently distinguish it are collected in the final claims accompanying this description.

Specifically, as mentioned above, the invention proposes a method for the electrochemical detection, identification, and quantification of inorganic nutrients dissolved in water. These nutrients include ions from nitrogen compounds such as ammonium/ammonia, nitrite, nitrate, and phosphorus-rich compounds such as phosphates and their chemical analogs. The voltammetric analysis and identification method of the invention allows the characterization of specific water masses, which may be of any type- including consumable, residual, urban, industrial, or natural resources. This evaluation is based on the application of a series of predefined and specific protocols for each ion or parameter, allowing for the specific production and interpretation of signals resulting from variations in an electrical current, caused by consecutive applications of positive and/or negative potentials to the sample within a range of -3,000 mV to 3,000 mV.

The method of the invention enables the detection and quantification of nutrients and other chemicals dissolved in water. Additionally, it can determine essential parameters such as pH and salinity in a single step using a potentiostat and a single type of sensor. This includes dissolved nitrogen compounds, such as ammonium (NH4), ammonia (NH3), nitrites (NO2), and nitrates (NO3), as well as phosphorus-rich compounds such as phosphates (PO4) and their chemical analogues at concentrations below 1 ppm (mg/L) and within a range of 0-100 ppm.

Furthermore, the method can determine essential parameters such as pH and salinity. The method is developed through an electro-analytical analysis that can also be applied to reusable screen-printed sensors (SPEs), which are cost-effective to replace.

In modern voltammetry, a time-dependent potential is applied to a working electrode. The current flowing between the auxiliary and working electrodes is measured relative to a reference electrode. Working electrodes are available in various materials, such as gold, silver, platinum, carbon, and mercury. The auxiliary electrode is typically made of platinum, and the reference electrode is made of silver/silver chloride.

Voltammetry is highly useful in combination with screen-printed electrodes (SPEs), making it suitable for portable systems, such as the one proposed in this invention. The reduction or oxidation of a substance on the surface of an electrode, under an appropriate potential, results in a current that is used in the unit to identify and quantify nutrients.

These and other objects, features, and advantages of the present invention will become more apparent from the following detailed description of the embodiments, the accompanying claims, and the appended drawings.

### BRIEF DESCRIPTION OF THE DRAWINGS

To complement the description being made and to aid in a better understanding of the characteristics of the invention, this descriptive report is accompanied, as an integral part of it, by a drawing in which, in an illustrative and non-limiting manner, the following has been represented:
Figure 1: Shows, in a diagram, the signals resulting from the application of the nitrate (NO3) measurement protocol to a series of water samples with different concentrations of this ion.
Figure 2: Shows, in another diagram, the signals resulting from the application of the phosphate (PO4) measurement protocol to a water sample with 1 ppm concentration of this ion and another sample free of phosphates.
Figure 3: Shows, in a block diagram, a schematic representation of the main components comprising the measurement equipment to carry out the detection method subject of the invention.

### PREFERRED EMBODIMENT OF THE INVENTION

The present method involves obtaining and interpreting molecular changes in the ions present in a water sample subjected to electrical stimulation through voltammetry. This is achievable because, under a specific positive or negative potential applied using a potentiostat, the "excited" molecules within the sample will ascend or descend to another stable energy level, known as oxidation or reduction. This process releases a certain amount of energy into the medium, which can then be measured as a current fluctuation. Because each ion or functional group of a molecule oxidizes or reduces at a specific potential under certain circumstances (e.g., pH), this enables, through a specific protocol (included in specially developed software with algorithms to achieve this goal), the evaluation of the sample to determine the analyte abundance or the physicochemical value under assessment.

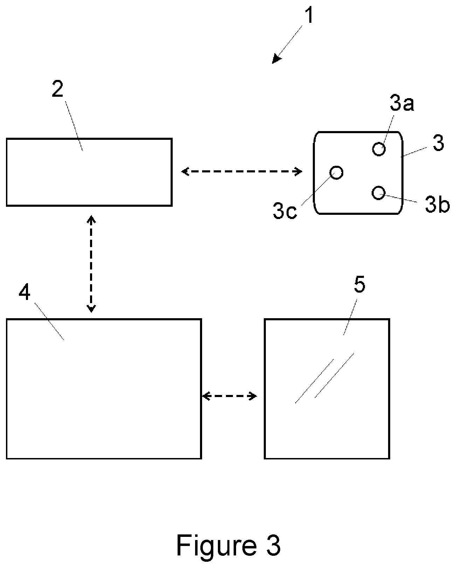

Thus, the method requires an analyzer device (1) that includes at least one potentiostat device (2) and one or more sensors (3) positioned in contact with the sample and interconnected with the potentiostat (2). Additionally, the device (1) will require the application of the methodology created through specific software developed for this purpose, whose execution requires a small computing system (4) to control stimulation, read signals, and interpret them based on the protocols described below, as well as a display unit (5), such as a touchscreen, necessary for output and managing the device as the user interface with the computing system (4). Preferably, the sensor (3) or sensors will be based on three electrodes: a reference electrode (3a), a working electrode (3b), and a counter (3c) or auxiliary electrode. The main elements of this device (1) are represented in Figure 3 through a block diagram.

The method focuses on identifying and quantifying inorganic ions dissolved in water that are rich in nitrogen (N) and phosphorus (P). These are chemical species highly involved in biological activity, such as dissolved residues/nutrients continuously produced, consumed, and transformed by living organisms, including phosphorus-rich chemical species like phosphates (PO4) and their analogs, and nitrogen-rich species like ammonium (NH4), ammonia (NH3), nitrite (NO2), and nitrate (NO3). The method also allows the measurement of certain parameters such as pH, REDOX potential, and water conductivity, as well as values derived from these, such as TDS (Total Dissolved Solids), degrees of water hardness, or density (which requires an additional temperature value).

Finally, the method can also be applied to identifying and quantifying other specific ions or salts such as potassium (K), calcium (Ca), magnesium (Mg), as well as dissolved metals like iron (Fe), cadmium (Cd), copper (Cu), lead (Pb), or zinc (Zn).

To perform the analysis, the method for detecting, identifying, and quantifying analytes, such as ions and nutrients dissolved in water through voltammetry, is based on interpreting the signal obtained from one or more consecutive electrochemical stimuli and comprises the following steps:
- A stimulation step involving the application, controlled via pre-programmed software in the computing system (4) connected to the potentiostat (2), of at least one electrical stimulation (potential) to the sample through Cyclic Voltammetry, Normal Pulse Voltammetry, Differential Pulse Voltammetry, or preferably Square Wave Voltammetry, within a potential range of -3,000 mV to 3,000 mV, pulse amplitudes between 0.1 and 250 mV, and frequencies between 1 Hz and 100 Hz. That is, the information contained within the specific software will ensure that an electrical stimulation or sequence of stimulations is applied to the sample. This stimulation consists of one or more consecutive pulses of positive and/or negative potentials within a voltage range of -3,000 mV to 3,000 mV, and these values can be restricted to more specific ranges depending on the nutrient type to be analyzed.
- A characterization step through the mentioned software in the computing system (4), characterizing one or more analytes within the analysis solution after applying at least one potential, based on at least one measurement of the signals resulting from the oxidation-reduction of the analyte(s) within the corresponding range. In this step, the software interprets the signals resulting from the oxidation/reduction reaction of the samples after each stimulation, resulting in an approximation of the analyte abundance value or the parameter value under assessment.

Preferably, the method of the invention includes one or more of the following steps:
- A preparation/placement step of a sensor (3) with at least one reference electrode (3a), one working electrode (3b), and one counter or auxiliary electrode (3c) in a potentiostat (2) capable of performing electrochemical analysis.
- A step involving bringing a sample or analysis solution containing the analyte into contact with the sensor (3). The sample must be added to the sensor (3) to contact the three electrodes (e.g., printed electrodes: a reference electrode (3a), a working electrode (3b), and a counter electrode (3c)), allowing electrical transfer and resulting reactions due to the electrical stimulation.

It is worth noting that the electrical circuit components and/or electrode surfaces of the sensor (3) used in the device to carry out the described method, namely the three-electrode system, including at least one reference electrode (3a), one working electrode (3b), and one counter electrode (3c) or auxiliary electrode, which may consist of a printed circuit, can be partially or entirely made of various materials based on carbon, gold, platinum, silver, or mercury and may or may not be coated with a layer of reagent to facilitate the reaction or increase the resulting signal.

On the other hand, the potentiostat (2) or electronic device in the analyzer device (1) required to control a cell or sensor (3) with three electrodes (3a, 3b, 3c) and execute electro-analytical experiments can consist of a potentiostat/galvanostat, a bi-potentiostat, or a poly-potentiostat.

Moreover, the sample containing the analyte or mixture of analytes, placed in a sensor (3) equipped with reference electrodes (3a), working electrodes (3b), and counter or auxiliary electrodes (3c), can be a pure water sample or one with a variable content of salts and ions, i.e., with variable salinities and hardness levels, or it can be a water-based extract representative of a sample, such as soil, organic fluids, or other material.

In another aspect of the invention and in accordance with the stimulation phase for analysis, as previously mentioned, one or a combination of different voltammetry methods can be applied, including Linear Sweep Voltammetry, Cyclic Voltammetry, AC Voltammetry, Normal Pulse Voltammetry, Differential Pulse Voltammetry, or Square Wave Voltammetry, with a scan potential range of -3,000 mV to 3,000 mV. The frequencies used for the analysis range from 1 Hz to 100 Hz..

Preferably, the stimulation phase of the method comprises several actions. First, before performing any stimulation phase or action, a continuous potential ranging from -3,000 mV to 3,000 mV can be applied for one or more seconds, referred to as "pre-treatment" or preparatory cycle. Furthermore, prior to the sweep or pulse analysis cycle, the potential can stabilize for one or more seconds at the initial potential of the sweep or pulse analysis, referred to as the "equilibrium time."

According to another aspect of the invention and depending on the stimulation and characterization phases, after initiating the stimulation method by applying a sweep or pulsed potential, the software will read the resulting current peaks generated during the oxidation/reduction process of the analyte. To this end, special algorithms designed to "find peaks" can modulate the sensitivity range, allowing signals to be omitted or included based on their intensity.

Finally, the software will interpret, based on the resulting signals (peaks), their absence, intensity, or location, the detection, identification, and possible conclusive quantification of the analytes.

In a preferred embodiment, for the detection, identification, and quantification of ions and nutrients dissolved in water, particularly inorganic nutrients rich in nitrogen in the form of **ammonium/ammonia** or **nitrite,** the stimulation phase of the method involves applying at least one electrical stimulation (potential) to the sample using Cyclic Voltammetry, Normal Pulse Voltammetry, Differential Pulse Voltammetry, or preferably Square Wave Voltammetry, with a step potential range for the sweep cycle of 0 mV to 2,500 mV.

Preferably, pre-treatment is carried out at low potential values ranging from -1,000 mV to 1,000 mV for one or more seconds; the equilibrium time may range from one to several seconds; and, for the sweep or analysis cycle, a frequency of 1 Hz to 20 Hz is applied, with an amplitude range of 0 to 100 mV.

The characterization phase, in turn, will comprise characterizing one or more analytes within the analysis solution after applying at least one potential, based on at least one measurement of the signals resulting from the oxidation-reduction of the analyte(s) within a range of 0 mV to 2,000 mV.

In another embodiment of the invention method, for the detection, identification, and quantification of ions and nutrients dissolved in water, particularly inorganic nutrients rich in nitrogen in the form of **nitrate,** the stimulation phase of the method involves applying at least one electrical stimulation (potential) to the sample using Cyclic Voltammetry, Normal Pulse Voltammetry, Differential Pulse Voltammetry, or preferably Square Wave Voltammetry, with a step potential range for the sweep cycle of 0 mV to 2,500 mV.

Preferably, pre-treatment is carried out at potential values ranging from - 2,000 mV to 2,000 mV for one or more seconds, the equilibrium time is one or several seconds, and, for the sweep or analysis cycle, a frequency of 1 Hz to 20 Hz is applied, with an amplitude range of 0 to 100 mV.

In this case, the characterization phase involves characterizing one or more analytes within the analysis solution after applying at least one potential, based on at least one measurement of the signals resulting from the oxidation-reduction of the analyte(s) within a range of 0 mV to 2,000 mV.

In another embodiment of the invention method, for the detection, identification, and quantification of ions and nutrients dissolved in water with high salinity above 1 PSU, particularly inorganic nutrients rich in nitrogen in the form of nitrate, the characterization phase of the method involves applying at least one electrical stimulation (potential) to the sample using Cyclic Voltammetry, Normal Pulse Voltammetry, Differential Pulse Voltammetry, or preferably Square Wave Voltammetry, with a step potential and pulse amplitude range of -2,500 mV to 0 mV.

Preferably, pre-treatment is carried out at potential values ranging from - 2,000 mV to 2,000 mV for one or more seconds, the equilibrium time is one or several seconds, and, for the sweep or analysis cycle, a frequency of 1 Hz to 20 Hz is applied, with a step potential and amplitude range of 0 to 100 mV.

In this case, the characterization phase involves characterizing one or more analytes within the analysis solution after applying at least one potential, based on at least one measurement of the signals resulting from the oxidation-reduction of the analyte(s) within a range of -2,000 mV to 0 mV.

Finally, in another embodiment of the invention method, for the detection, identification, and quantification of ions and nutrients dissolved in water, particularly inorganic **nutrients rich in phosphorus such as phosphates,** the stimulation phase involves providing at least one electrical stimulation (potential) to the sample using Cyclic Voltammetry, Normal Pulse Voltammetry, Differential Pulse Voltammetry, or preferably Square Wave Voltammetry, with a step potential range for the sweep cycle of 0 mV to 2,000 mV. In this case, preferably, pre-treatment is carried out at potential values ranging from -3,000 mV to 3,000 mV for one or more seconds, the equilibrium time is one or several seconds, and, for the sweep or analysis cycle, a frequency of 0 Hz to 10 Hz is applied, with a step potential and amplitude range of 0 to 100 mV.

In turn, the characterization phase will comprise characterizing one or more analytes within the analysis solution after applying at least one potential, based on at least one measurement of the signals resulting from the oxidation-reduction of the analyte(s) within a range of 0 mV to 2,000 mV.

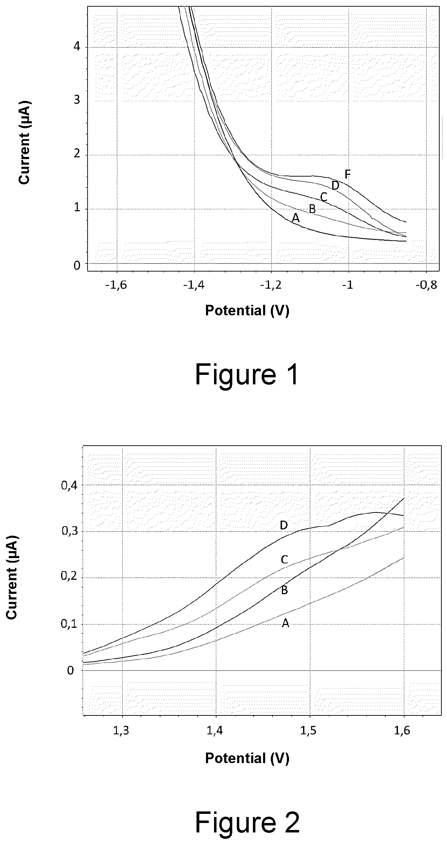

Lastly, referring to Figures 1 and 2, two Cartesian diagrams show the signals resulting from applying the invention method in two examples of its implementation, where the X-axis or horizontal coordinate indicates potential values in volts and the Y-axis or vertical coordinate indicates current values in microamperes.

## Claims

1. An electrochemical method for detecting, identifying, and quantifying analytes consisting of inorganic nutrients rich in nitrogen in the form of ammonium/ammonia or nitrite dissolved in a water sample through voltammetry, **characterized by** comprising:
• a pre-treatment stage by applying a continuous potential between - 1,000 mV and 1,000 mV for one or more seconds;
• an equilibrium stage lasting one or more seconds;
• an electrical stimulation stage of the sample by applying a sweep potential between 0 mV and 2,500 mV for one or more seconds at a frequency of 1 Hz to 20 Hz, with an amplitude range of 0 to 100 mV;
• a characterization stage of the analytes by measuring the signals resulting from the oxidation-reduction of the analytes within a range of 0 mV to 2,000 mV.

2. An electrochemical method for detecting, identifying, and quantifying analytes consisting of inorganic nutrients rich in nitrogen in the form of nitrate dissolved in a water sample through voltammetry, **characterized by** comprising:
• a pre-treatment stage by applying a continuous potential between - 2,000 mV and 2,000 mV for one or more seconds;
• an equilibrium stage lasting one or more seconds;
• an electrical stimulation stage of the sample by applying a sweep potential between 2,500 mV and 0 mV for one or more seconds at a frequency of 1 Hz to 20 Hz, with an amplitude range of 0 to 100 mV;
• a characterization stage of the analytes by measuring the signals resulting from the oxidation-reduction of the analytes within a range of 0 mV to 2,000 mV.

3. An electrochemical method for detecting, identifying, and quantifying analytes consisting of inorganic nutrients rich in nitrogen in the form of nitrate dissolved in water with high salinity exceeding 1 PSU in a sample through voltammetry, **characterized by** comprising:
• a pre-treatment stage by applying a continuous potential between - 2,500 mV and 2,500 mV for one or more seconds;
• an equilibrium stage lasting one or more seconds;
• an electrical stimulation stage of the sample by applying a sweep potential between -2,500 mV and 0 mV for one or more seconds at a frequency of 1 Hz to 20 Hz, with an amplitude range of 0 to 100 mV;
• a characterization stage of the analytes by measuring the signals resulting from the oxidation-reduction of the analytes within a range of -2,000 mV to 0 mV.

4. An electrochemical method for detecting, identifying, and quantifying analytes consisting of inorganic nutrients rich in phosphorus such as phosphates in a sample through voltammetry, **characterized by** comprising:
• pre-treatment stage by applying a continuous potential between - 3,000 mV and 3,000 mV for one or more seconds;
• an equilibrium stage lasting one or more seconds;
• an electrical stimulation stage (potential) of the sample by applying a sweep potential between 0 mV and 2,000 mV for one or more seconds at a frequency of 1 Hz to 10 Hz, with an amplitude range of 0 to 100 mV;
• a characterization stage of the analytes by measuring the signals resulting from the oxidation-reduction of the analytes within a range of 0 mV to 2,000 mV.

5. An electrochemical method for detecting, identifying, and quantifying analytes, according to any of the preceding claims, **characterized in that**, after initiating the electrical stimulation phase, the software reads the resulting current peaks generated during the oxidation/reduction process of the analyte using special algorithms designed to find peaks that can modulate the sensitivity range, allowing signals to be omitted or included based on their intensity, and whereby, from the resulting signals (peaks), their absence, intensity, or location, the software interprets the detection, identification, and possible conclusive quantification of the analytes.

6. Equipment for carrying out the electrochemical method for detecting, identifying, and quantifying analytes, according to any of the preceding claims, **characterized by** comprising at least: a potentiostat device (2); a sensor (3) that can be placed in contact with the sample to be analyzed and interconnected with the potentiostat (2); a computer system (4) with specific software to control stimulation, signal reading, and interpretation; and a display unit (5) as a user interface.
